(19) Europäisches Patentamt
European Patent Office
Office européen des brevets

(11) **EP 2 120 720 B1**

(12) **EUROPEAN PATENT SPECIFICATION**

(45) Date of publication and mention
of the grant of the patent:
**30.11.2011  Bulletin 2011/48**

(21) Application number: **07849560.3**

(22) Date of filing: **11.12.2007**

(51) Int Cl.:
***A61B 7/00*** (2006.01)

(86) International application number:
**PCT/IL2007/001533**

(87) International publication number:
**WO 2008/072233 (19.06.2008 Gazette 2008/25)**

(54) **METHOD AND SYSTEM FOR ANALYZING BODY SOUNDS**

VERFAHREN UND SYSTEM ZUR ANALYSE VON KÖRPERGERÄUSCHEN

PROCEDE ET SYSTEME POUR ANALYSER DES BRUITS DU CORPS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.12.2006  US 873945 P**

(43) Date of publication of application:
**25.11.2009  Bulletin 2009/48**

(73) Proprietor: **Deepbreeze Ltd.**
**Or Akiva 30600 (IL)**

(72) Inventors:
• **RADZIEVSKY, Naira**
  **33142 Haifa (IL)**
• **PAPYAN, Surik**
  **33074 Haifa (IL)**

(74) Representative: **Becker, Philippe**
**Cabinet Becker & Associés**
**25, rue Louis Le Grand**
**75002 Paris (FR)**

(56) References cited:
WO-A-03/071952      WO-A-2004/105612
WO-A-2005/074799    US-A1- 2003 130 588

• **KOMPIS M ET AL: "Acoustic Imaging of the Human Chest" CHEST, THE COLLEGE, CHICAGO, IL, US, vol. 120, no. 4, October 2001 (2001-10), pages 1309-1321, XP002237833 ISSN: 0012-3692**

**Description**

**FIELD OF THE INVENTION**

This invention relates to medical devices and methods, and more particularly to such devices and methods for analyzing body sounds.

**BACKGROUND OF THE INVENTION**

Body sounds are routinely used by physicians in the diagnosis of various disorders. A physician may place a stethoscope on a person's chest or back and monitor the patient's breathing or heartbeat in order to detect adventitious (i.e. abnormal or unexpected) lung or heartsounds. The identification and classification of adventitious lung or heart sounds often provides important information about pulmonary or cardiac abnormalities.

It is also known to fix one or more microphones onto a subject's chest or back and to record lung sounds. U.S. Patent No. 6,139,505 discloses a system in which a plurality of microphones are placed around a patient's chest. The recordings of the microphones during inhalation and expiration are displayed on a screen, or printed on paper. The recordings are then visually examined by a physician in order to detect a pulmonary disorder in the patent. Kompis et al. (Chest, 120(4), 2001) disclose a system in which M microphones are placed on a patient's chest, and lung sounds are recorded. The recordings generate *M* linear equations that are solved using a least-squares fit. The solution of the system is used to determine the location in the lungs of the source of a sound detected in the recordings.

US Patent No. 6,887208A discloses method and system for analyzing respiratory tract sounds in which sound transducers are fixed over the thorax. Each transducer generates a signal indicative of pressure waves arriving at the transducer. A processor determines, for each signal, an average acoustic energy in the signal over time intervals. The acoustic energies are used to generate an image of the lungs.

International application WO2004/105612A discloses method and system for analyzing sounds originating in at least a portion of an individual's cardiovascular system. Sound transducers are fixed over the thorax. Each transducer generates a signal indicative of pressure waves arriving at the transducer which are processed to generate filtered signals in which components of the signals not arising from cardiovascular sounds are removed. The filtered signals may be used for generating an image of the at least portion of the cardiovascular system

**SUMMARY OF THE INVENTION**

In the following description and set of claims, two explicitly described, calculable, or measurable variables are considered equivalent to each other when the two variables are proportional to one another.

The present invention provides a method and system for recording and analyzing heart sounds. The system of the invention comprises one or more sound transducers that are applied to a region overlying the body organ or organs from which sounds are to be recorded and analyzed. Each transducer produces a time signal indicative of the pressure wave arriving at the location on the skin surface of the transducer. The sound signals are filtered to in order to remove one or more components of the signals not arising from the body organ or organs whose sounds are to be analyzed. For example, if cardiovascular signals are to be recorded and analyzed then the signals should be filtered in order to remove respiratory tract sounds. Each filtered signal is divided into time intervals using a time window, and the average value of the signal in each interval is calculated. A difference signal is then calculated in each interval as the difference between the filtered signal and the signal average in the interval. An energy assessment signal is then calculated from the difference signal. In a presently preferred embodiment, the standard deviation of each signal in each interval is calculated from the difference signals that is used to calculate the energy assessment signal. In a presently preferred embodiment, the standard deviation signals are first processed by normalization, filtering and denoising, as explained below. The processed standard deviation signal then is divided into subintervals and the energy assessment signal is calculated as the sum of squares of the processed standard deviation signal.

As shown below, the energy assessment signal of the present invention may be used to diagnose abnormal heart function, and to calculate cardiac ejection fraction.

It will also be understood that the system according to the invention may be a suitably programmed computer. Likewise, the invention contemplates a computer program being readable by a computer for executing the method of the invention. The invention further contemplates a machine-readable memory tangibly embodying a program of instructions executable by the machine for executing the method of the invention.

Thus, in one of its aspects, the invention provides a system for analyzing body sounds from one or more body organs, comprising:

(a) An integer N of transducers, each transducer configured to be fixed on a region of a surface of the individual, a transducer $i$ being fixed at a location $x_i$ and generating a signal $S(x_i,t)$ indicative of pressure waves at the location $x_i$; for i=1 to N; and

(b) a processor receiving the signals $S(x_i,t)$ configured, for each of one or more of the signals $S(x_i,t)$ and for each of one or more time intervals k, to:

(a) calculate an average $\overline{S}_k$ of the signal $S(x_i,t)$ in the interval k; and

(b) for one or more times $t_j$ in the interval k, calculate a difference $S(x_i,t_j)$- $\overline{S}_k$ ; and

(c) calculate an energy assessment signal in a calculation involving the one or more differences $S(x_i,t_j)$- $\overline{S}_k$.

In another of its aspects, the invention provides a method for analyzing body sounds from one or more body organs, comprising:

(c) affixing an integer N of transducers on a region of a surface of the individual, a transducer $i$ being fixed at a location $x_i$, each tranducer and generating a signal $S(x_i,t)$ indicative of pressure waves at the location $x_i$; for i=1 to N; and

(d) for each of one or more of the signals $S(x_i,t)$ and for each of one or more time intervals k:

(a) calculating an average $\overline{S}_k$ of the signal $S(x_i,t)$ in the interval k; and

(b) for one or more times $t_j$ in the interval k, calculating a difference $S(x_i,t_j)$- $\overline{S}_k$; and

(c) calculating an energy assessment signal in a calculation involving the one or more differences $S(x_i,t_j)$- $\overline{S}_k$.

In still another of its aspects, the invention provides a system for determining an ejection fraction of a heart chamber, comprising:

(a) An integer N of transducers, each transducer configured to be fixed on a surface of the individual over the thorax, an ith transducer being fixed at a location $x_i$ and generating a signal $S(x_i,t)$ indicative of pressure waves at the location $x_i$; for i=1 to N; and

(a) a processor configured to receive the signals $S(x_i,t)$ and to calculate the ejection fraction in a method including a calculation involving one or more of the signals $S(x_i,t)$.

In yet another of its aspects, the invention provides a method for determining an ejection fraction of a heart chamber, comprising:

(a) Affixing an integer N of transducers, each over the thorax, the ith transducer being fixed at a location $x_i$ and generating a signal $S(x_i,t)$ indicative of pressure waves at the location $x_i$; for i=1 to N; and

(b) calculating the ejection fraction in a method including a calculation involving one or more of the signals $S(x_i,t)$.

The invention also provides a computer program comprising computer program code means for performing all the steps of the method of the invention when said program is run on a computer, and the computer program embodied on a computer readable medium.

## BRIEF DESCRIPTION OF THE DRAWINGS

In order to understand the invention and to see how it may be carried out in practice, a preferred embodiment will now be described, by way of non-limiting example only, with reference to the accompanying drawings, in which:

**Fig. 1** shows a system for recording and analyzing body sounds in accordance with one embodiment of the invention;
**Fig. 2** shows a method for calculating an energy assessment signal from a difference signal in accordance with the invention;
**Fig. 3** shows a method for calculating an energy assessment signal from a standard deviation signal in accordance with the invention;
**Fig. 4** shows a method for calculating an energy assessment signal from a standard deviation signal as a sum of squares;
**Fig. 5a** shows a sound signal obtained by a microphone placed on the chest of an individual over the heart apex and an electrocardiogram obtained simultaneously, **Fig. 5b** shows a sound signal obtained simultaneously with the sound signal of Fig. 5a by a microphone placed on the back of the same individual, together with the electrocardiogram, and **Fig. 5c** shows the energy assessment signal calculated from the sound recordings of Fig. 5a and 5b, together with the electrocardiogram;

**Fig. 6** shows, for an individual with normal heart function, a cardiac sound signal **(Fig. 6a),** an electrocardiograph **(Fig. 6b)** and an energy assessment signal **(Fig. 6c),** in accordance with the invention;

**Fig. 7** shows, for an individual with abnormal heart function, a cardiac sound signal **(Fig. 7a),** an electrocardiograph **(Fig. 7b)** and an energy assessment signal **(Fig. 6c),** in accordance with the invention;

**Fig. 8** shows energy assessment signals obtained simultaneously from a 6X6 array of transducers placed on the back of an individual over the heart region;

**Fig. 9** shows an energy assessment signal of an individual having normal heart function (Fig. 9a**)** and an individual having abnormal heart function (Fig. 9b) at the time of the sound event E1;

**Fig. 10** shows the energy assessment signal of an individual having normal heart function at E1 **(Fig. 10a),** E2 **(Fig. 10b),** E3 **(Fig. 10c)** and E4 **(Fig. 10d);**

**Fig. 11** shows the energy assessment signal of an individual having abnormal heart function at E1 (Fig. **11a**), E2 (Fig. **11b**), E3 (Fig. **11c**) and E4 (Fig. **11d**);

**Fig. 12** shows a method for identifying transducers in a transducer array that overlie the heart apex; and

**Fig. 13** shows a scatter plot of cardiac ejection fraction, as determined by the method of the invention as a function of cardiac ejection fraction as determined by echocardiography.

## DETAILED DESCRIPTION OF THE INVENTION

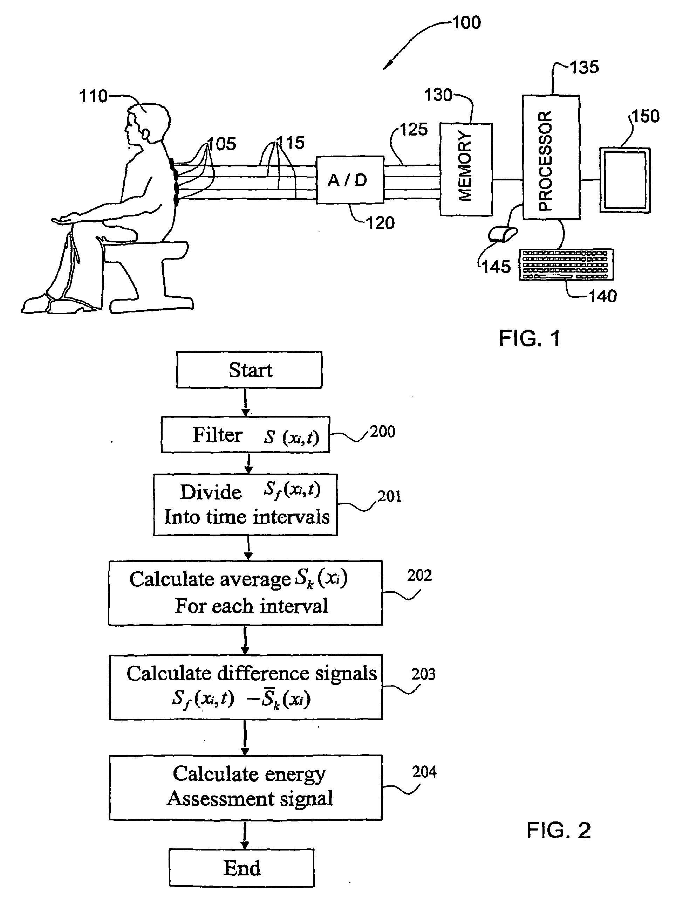

Fig. 1 shows a system generally indicated by **100** for recording and analyzing body sounds in accordance with one embodiment of the invention. One or more sound transducers **105** are applied to a planar region R of the skin surface of an individual **110.** Four transducers, **105a, 105b, 105c,** and **105d,** are shown in Fig. 1. This is by way of example only, and the any number of transducers may be used in the invention, as required in any application. Fig. 1 shows the transducers **105** being applied to a planar region overlying the back skin surface of the individual 110 as may be done, for example, to record and analyze respiratory or cardiovascular sounds. Positions in the region R are indicated by two-dimensional position vectors $x=(X^1,X^2)$ in a two-dimensional coordinate system defined in the planar region R. The ith transducer, for i=1 to N, is fixed at a position $x_i$ in the region R and generates a signal, denoted herein by s($x_i$, t), indicative of pressure waves in the body arriving at the location $x_i$. The transducers **105** may be any type of sound transducer, such as a microphone or a Doppler shift detector. The transducers **105** may be applied to the subject by any means known in the art, for example using an adhesive, suction, or fastening straps. Each transducer **105** produces a respective analog voltage signal **115** over a time interval that is indicative of pressure waves and arriving at the transducer. The analog signals **115** are digitized by an analog to digital converter **120** generating respective digital data signals $S(x_i, t)$ **125** indicative of the pressure wave at the location $x_i$ on the skin surface of the transducer at time t. The digital data signals **125** are input to a memory **130.** Data input to the memory **130** are accessed by a processor **135** configured to process the data signal **125.** An input device such as a computer keyboard **140** or mouse **145** is used to input relevant information relating to the examination such as personal details of the individual **110,** as well as any values of the parameters used in the signal processing.

Fig. 2 shows a method in accordance with one embodiment of the invention for analyzing any one of the signals $S(x_i, t)$ **125** which is carried out by the processor **135.** In step **200,** the signal $S(x_i, t)$ **125** is filtered to produce a filtered signal $S_f(x_i,t)$ in order to remove one or more components of the signal $S(x_i, t)$ which do not arise from the body organ or organs whose sounds are to be analyzed. For example, if cardiovascular signals are to be recorded and analyzed then the signals $S(x_i, t)$ **125** may be filtered in order to remove respiratory tract sounds. Cardiac sounds are typically in the range of 8 to 70 Hz, while respiratory tract sounds are in the range of 100 to 2000 Hz. Thus, respiratory tract sounds can be removed from the signal by band pass filtering in the rage of 10-66 Hz. This band pass filtering also removes from the signals artifacts and adventitious lung sounds.

Alternatively or additionally, if cardiac sounds are to be recorded and analyzed, the subject 110 may be instructed not to breath during recording of the cardiac sounds.

In step **201,** the filtered signal $S_f(x_i,t)$ is divided into time intervals using a sliding time window, and the average value $\overline{S}_k(x_i)$ of the signal in each interval is calculated, where

$$\overline{S}_k(x_i) = \frac{1}{n}\sum_{j=1}^{nk} S_f(x_i, t_j) \qquad (1)$$

wherein $k$ is the interval number, $t_j$ is a time sample in the interval, and $n_k$ is the number of samples in the interval. In step **203,** a difference signal $S_f(x_i,t)-\overline{S}_k(x_i)$ is calculated for each of the $n_k$ intervals. In step **203,** an energy assessment signal is calculated in a calculation involving the difference signals $S_f(x_i,t)-\overline{S}_k(x_i)$, k=1 to $n_k$.

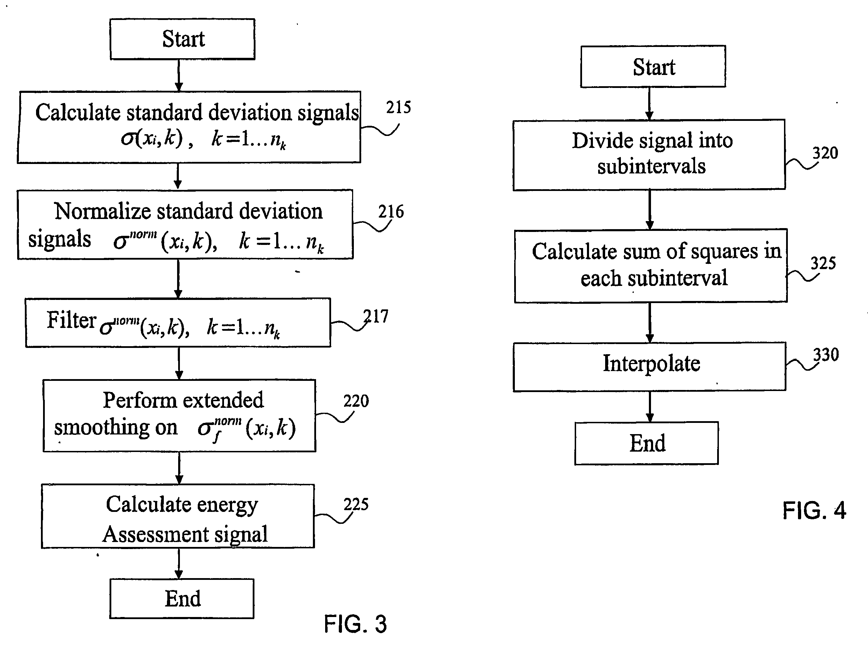

The calculation of the energy assessment signal may involve the algebraic expression $|S_f(x_i,t)-\overline{S}_k(x_i)|$ or the

expression $(S_f(x_i,t)-\overline{S}_k(x_i))^p$ where p is a predetermined constant. In a presently preferred embodiment, p=2. In an even most preferred embodiment, as described below, the energy assessment signal is calculated using the standard deviation of the signals $S_f(x_i,t)$ in each interval k. Fig. 3 shows a method for calculating an energy assessment signal in step **204** (Fig. 2) in accordance with this embodiment. In step **215**, a standard deviation signal $\sigma(xi,k)$ is obtained from the filtered signal $S_f(xi,t)$ obtained in step **200**. To obtain a standard deviation signal, the signal $S_f(xi,t)$ is divided into time intervals and the standard deviation $\sigma(xi,k)$ for each interval is calculated, where

$$\sigma(k) = \left(\frac{1}{n}\sum_{j=1}^{n}\left(S_f(t_j)-\overline{S}_k\right)^2\right)^{\frac{1}{2}}, \qquad k=1 \ to \ n_k. \tag{2}$$

A normalized standard deviation vector $\sigma^{norm}(x_i,k)$ is then calculated in step **216**, where

$$\sigma^{norm}(xi,k) = \sigma(xi,k)/\overline{\sigma}(xi), \quad k=1...n_k$$

where $\overline{\sigma}(x_i)$ is the average value of the standard deviation calculated for all of the intervals:

$$\overline{\sigma}(xi) = \frac{1}{n_k}\sum_{k=1}^{n_k}\sigma(xi,k) \tag{3}$$

Next, in step 217, the $\sigma^{norm}(x_i,k)$, for $k=1$ to $n_k$ are preferably filtered. The filtering is preferably a one-dimensional median filtering, for example, as performed by using the MATLAB algorithm "*midfield1*". This generates a filtered normalized standard deviation vector $\sigma_f^{norm}(xi,k)$. In step **220** extended smoothing is preferably performed on the vector $\sigma_f^{norm}(xi,k)$, for example, using the MATLAB algorithm "*sgolayfilt*", which implements the so-called Savitzky-Golay smoothing filter. This produces a filtered and smoothed normalized sequence $\sigma_{fs}^{norm}(xi,k)$. This tends to remove impulse artifacts ("*clicks*") introduced into the signal by ambient noise.

In step **225**, an energy assessment signal is calculated at a sequence of time points over the time interval from the filtered and smoothed normalized sequence $\sigma_{fs}^{norm}(xi,k)$. Any method for calculating an energy assessment signal from the filtered and denoised signals $\sigma_{fs}^{norm}(xi,k)$ may be used in step **225** of the algorithm of Fig. 3. Fig. 4 shows a presently preferred method for calculating an energy assessment signal from the filtered and denoised signal $\sigma_{fs}^{norm}(xi,k)$ $k=1$ to $n_k$. In step **320**, the $n_k$-dimensional vector $\sigma_{fs}^{norm}(xi,k)$ is divided into subintervals by a sliding window having $n_s$ samples. In step **325**, an energy assessment signal in the interval $k_s$, $P(k_s)$, is calculated as the sum of squares of $\sigma_{fs}^{norm}(k_s)$:

$$P(xi,k_s) = \sum_{l=1}^{n_s}\left[\left(\sigma_{fs}^{norm}(xi,l)\right)^2\right]_{k_s} \tag{4}$$

where $k_s$ is the interval number and $l$ belongs to this interval. In step **330**, the energy assessment signals $P(xi,k_s)$ are interpolated in the position variable x in order to provide energy assessment signals at locations in the region R between microphones in the microphone array.

**Examples**

Example 1: Calculation of an energy assessment signal

The heart sounds are produced by the vibrations of the cusps and ventricles when the upward movement of the cusps' bellies is suddenly checked at the crossover of pressure pulses in the ventricles and atria. Heart sounds are traditionally recorded using a microphone placed on the individual's chest over the heart apex. Two heart sounds have been defined that are detectable in cardiac sound signals recorded on the chest over the heart apex.

One heart sound, known as **"S1"**, is caused by turbulence caused by the closure of the mitral and tricuspid valves and aortic valve opening at the start of systole between the times of the S and T points of the ECG signal. A second heart sound, known as "*S2*" is caused by the closure of the aortic and pulmonary valves at the end of systole after the T wave.

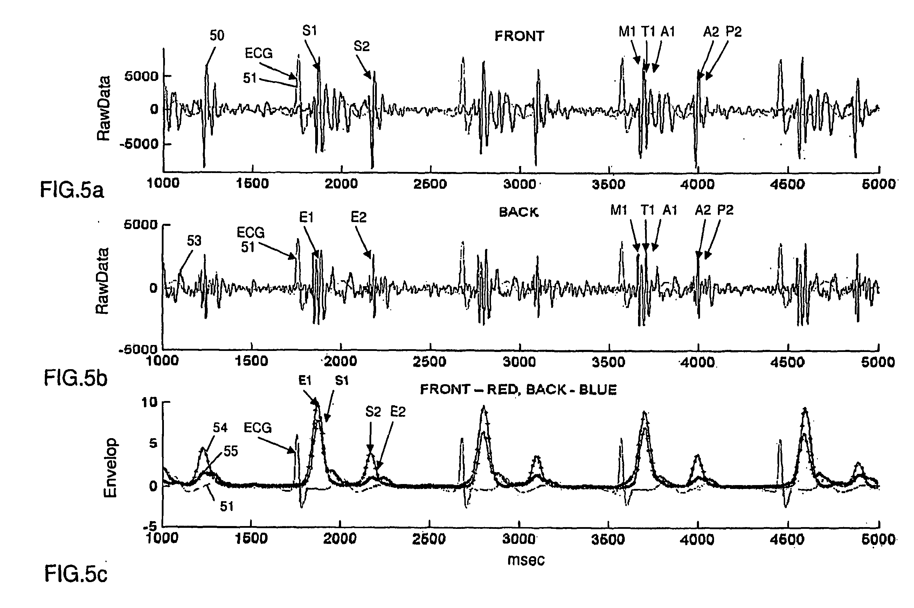

Fig. 5a shows a sound signal **50** obtained by a microphone placed on the chest of a healthy individual over the heart apex, together with the electrocardiogram **51** that was recorded simultaneously. The heart sounds **S1** and S2 are observed in the recorded sound signal of Fig. 5a.

Fig. 5b shows a sound signal **53** obtained simultaneously with the sound signal **50** of Fig. 5a by a microphone placed on the back of the same individual over the heart apex, together with the electrocardiogram **51.** Two events of the cardiovascular cycle, referred to herein as **"E1"** and **"E2"** are observed in the sound signal of Fig. 5b. The sound event **E1** occurs at about the same time as the sound event **S1** observed in the chest recording (Fig. 5a). The sound event **E2** occurs at about the same time as the sound event S1 observed in the chest recording (Fig. 5a).

Fig. 5c shows energy assessment signals **54** and **55** calculated according to Equation (4) above from the acoustic signals in Figs. 5a and 5b, respectively, together with the ECG **51.** In the acoustic energy signals of the chest signal, the heart sounds S1 and S2 are observed as a peak **S1** and a smaller peak **S2**. In the acoustic energy signals of the back signal, the sound events E1 and E2 are observed as a peak **E1** and a peak **E2.**

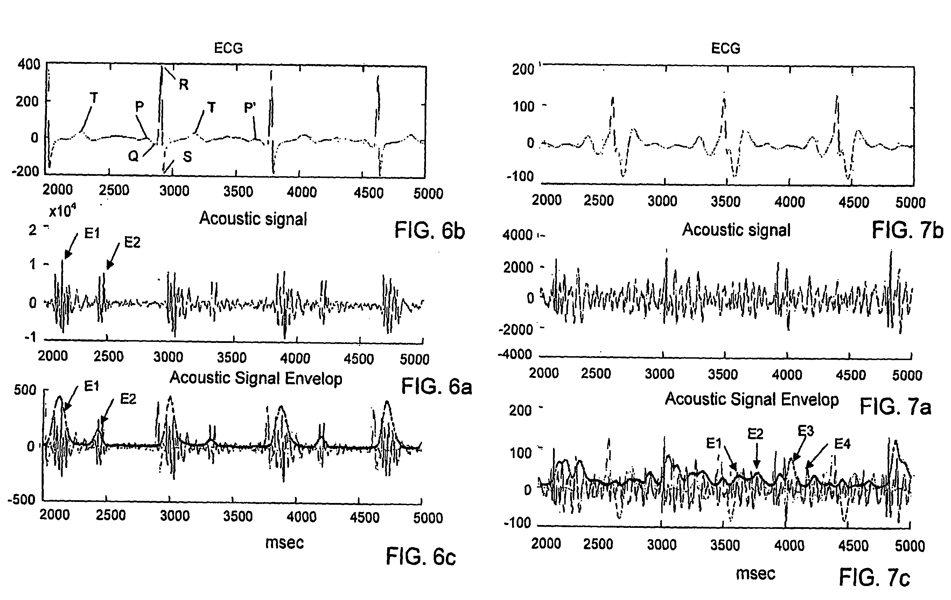

Fig. 6a shows a sound signal $S(x_i,t)$ obtained by a microphone placed on the back of a healthy individual with normal heart function over the apex region of the heart, as explained above. Fig. 6b shows the electrocardiogram that was obtained simultaneously with the sound signal. A typical cardiac cycle of the ECG signal includes a P wave **P,** a **QRS** complex, a **T** wave, and terminates with the P wave **P'** of the next cardiac cycle. In the acoustic signal shown in Fig. 6a, the acoustic events E1, and E2 are observed. Fig. 5c shows the energy assessment signal calculated according to Equation (4) above, in which the events E1 and E2 are observed as a peak, and a smaller peak, respectively, in the energy assessment signal.

Fig. 7a shows a sound signal obtained by a microphone placed on the back of an individual at a location xi over the apex region of the heart. Fig. 7b shows the electrocardiogram that was obtained simultaneously with the sound signal. Fig. 7c shows the energy assessment signal calculated according to Equation (6) above. The individual has an abnormal heart function. The events E1 and E2 are less prominent in the acoustic signal of Fig. 7a and energy assessment signal of Fig. 7b, in comparison to those in the acoustic signal of Fig. 6b and the energy assessment signal of Fig. 6c. On the other hand, two acoustic events E3 and E4, are observed acoustic signal of Fig. 6b or in the acoustic assessment signal of Fig. 7c that are not observed in the energy assessment signal of Fig. 6c. The acoustic events E3 and E4 correspond in time to heart sounds known as "*S3*" and "*S4*" that have been defined in heart sound recordings obtained by a microphone placed on the chest over the heart apex. The heart sounds S3 and S4 are "gallops", which are low frequency sounds that are associated with diastolic filling. The gallop S3 is associated with early diastolic filling and may be heard pathologically in such states as volume overload and left ventricular systolic dysfunction. The gallop S4 is a late diastolic sound and may be heard in such pathologic states as uncontrolled hypertension. The acoustic assessment signal can thus be used to diagnose abnormal heart function. If the magnitude of the energy assessment signal at the time of E1 and/or E2 is below a predetermined first threshold, or if the magnitude of the energy assessment signal at the time of E3 and/or E4 is above a second predetermined constant, then the individual has abnormal heart function.

Example 2: Acoustic Energy in a plurality of cardiac signals

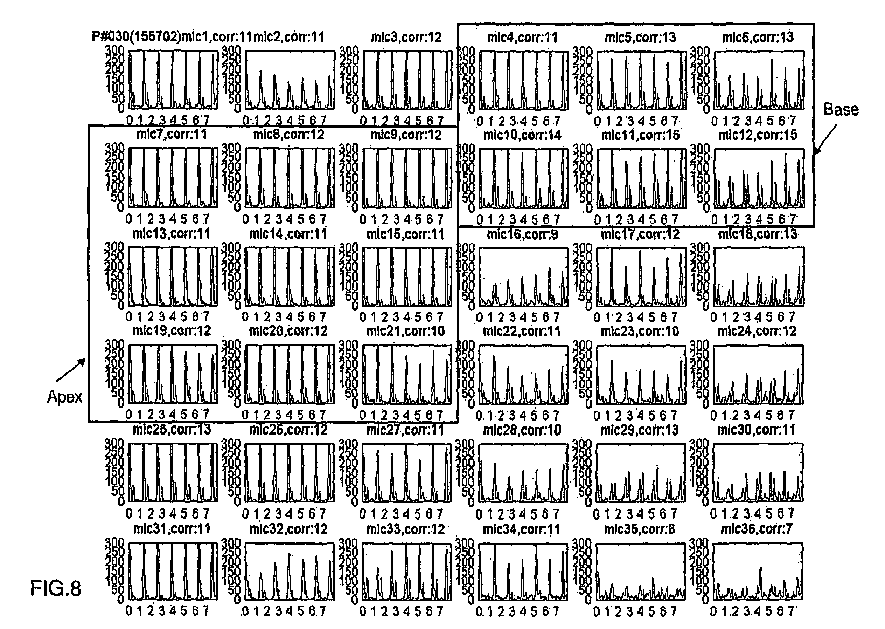

A 6X6 array of sound transducers was affixed to the back of individuals over the cardiac region in order to record and analyze cardiovascular sounds. The transducers had a center to center spacing in the array of 3.5 cm. For each transducer in the array, an energy assessment signal was calculated from the transducer sound signal using Equation (4) above. Fig. 8 shows the energy assessment signal for each of the 36 transducers over approximately 6 cardiac cycles obtained on a healthy individual.

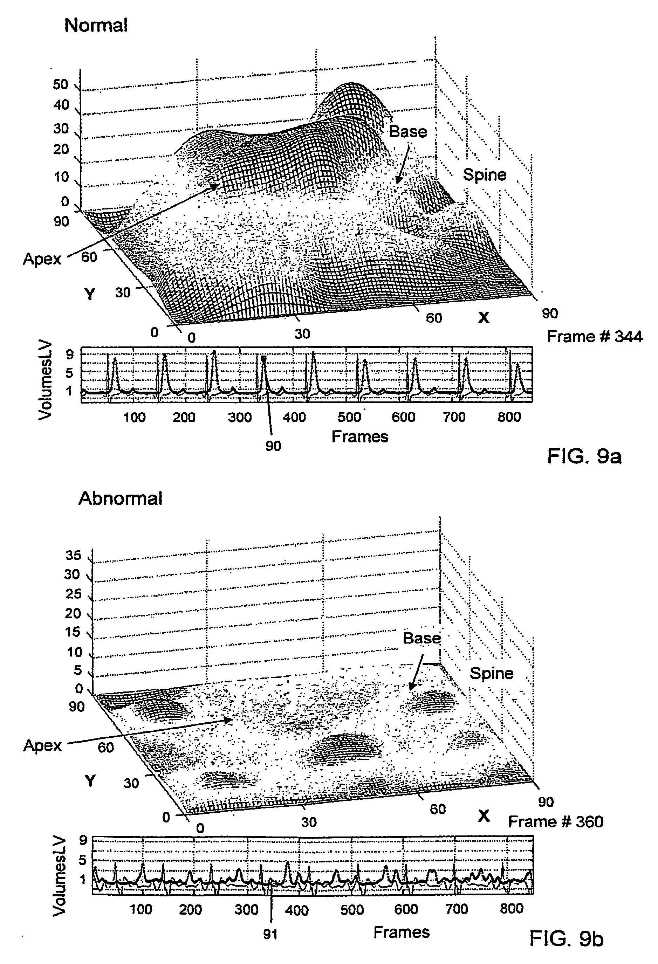

Fig. 9 shows the of the interpolated energy assessment signals over the region of the microphone array at the time of the event E1 for an individual with normal heart function (Fig. 9a) and for an individual with abnormal heart function (Fig. 9b). The X and Y axis of the graphs indicate the location in the region. The region of the interpolated energy assessment function overlying the heart apex is indicated. In the region of the heart apex, the energy assessment signal is substantially greater for the individual with normal heart function than for the individual with abnormal heart function.

Figs. 9a and 9b also show an ECG of the individuals obtained simultaneously with the acoustic sound recordings from which the acoustic energy signals of Figs 9a and 9b were obtained. The time during the cardiac cycle of the respective energy assessment signal is indicated by a dot **90** and **91** in the ECG.

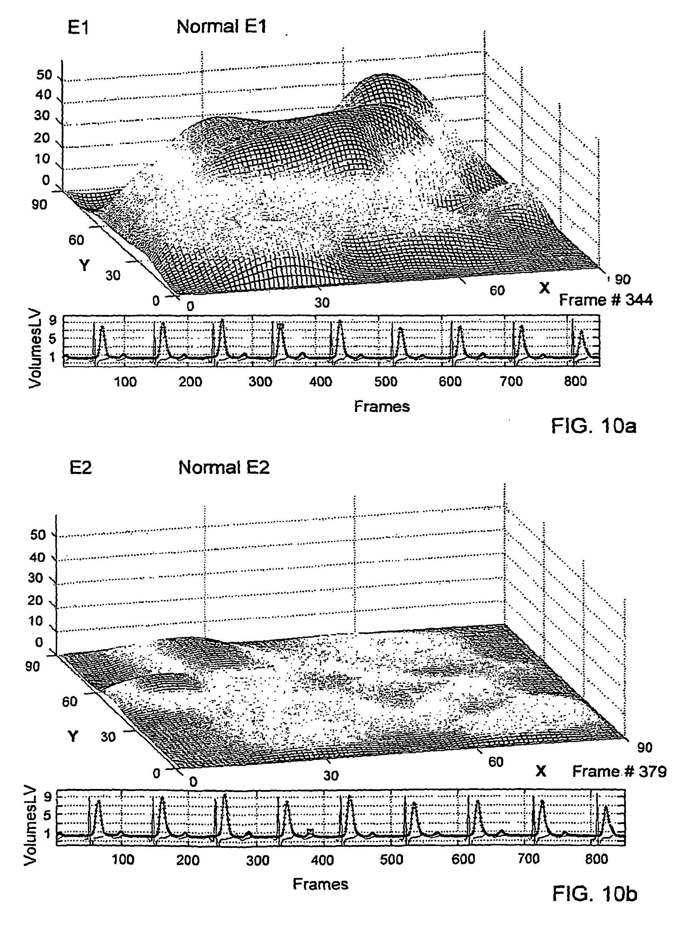

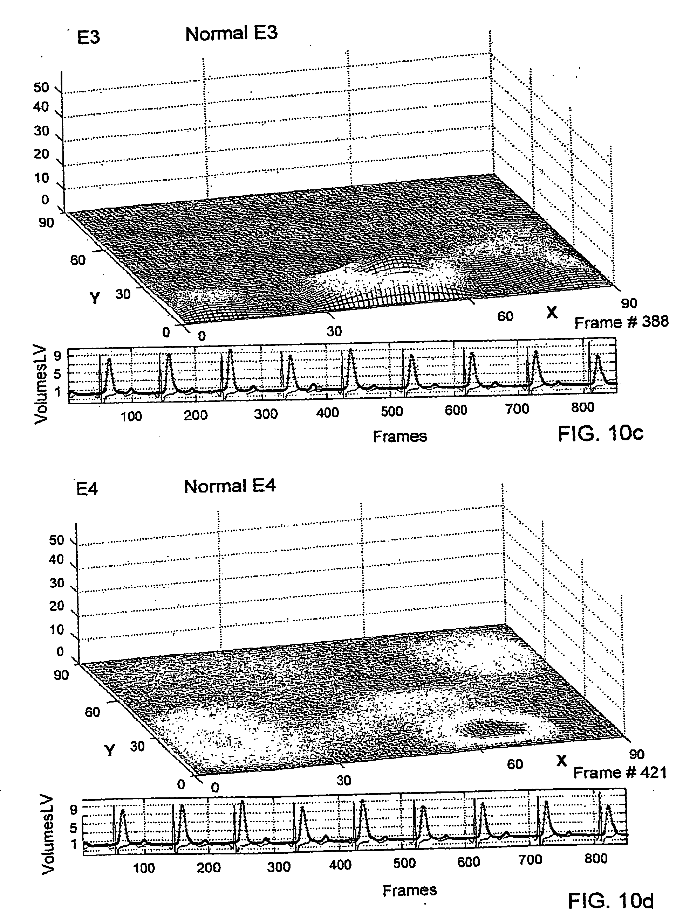

Fig. 10 shows the value of the interpolated energy assessment signals over the region of the microphone array at four times during the cardiac cycle. The X and Y axis of the graphs indicate the position in the region. The graph in Fig. 10a shows the energy assessment signal at the time of E1. The graph in Fig. 10b shows the energy assessment signal at the time of E2, the graph in Fig. 10c shows the energy assessment signal at the time of E3, and the graph in Fig. 10d shows the value of the energy assessment signals of the microphone array at the time of E4.

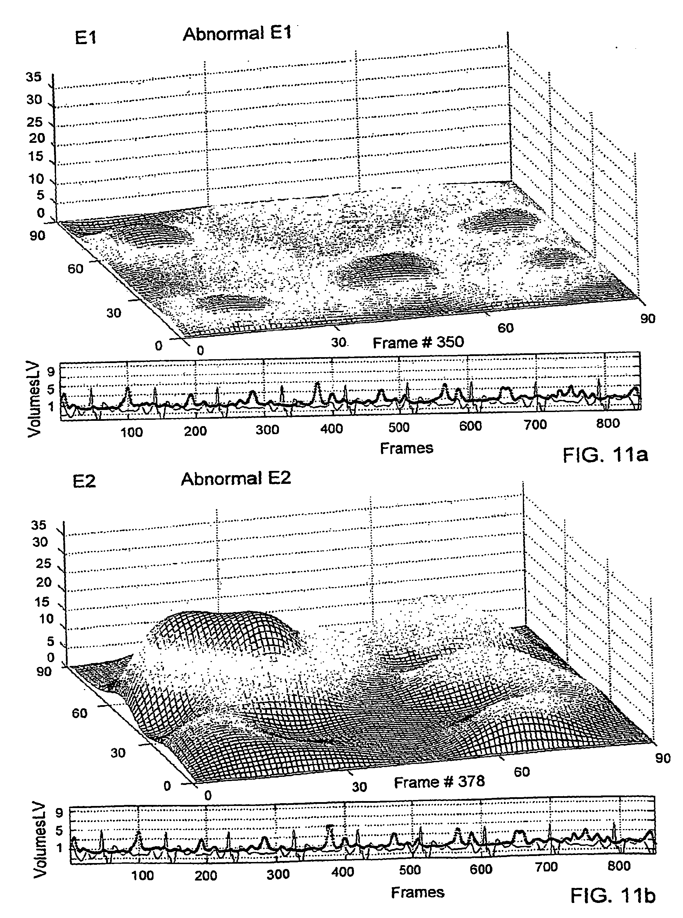

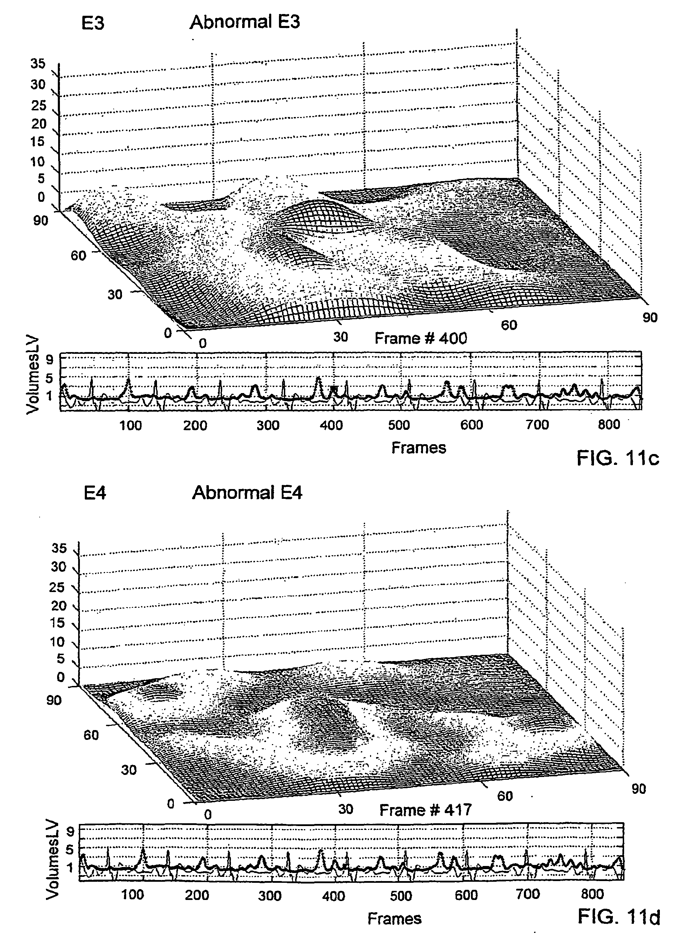

Figs. 11a to 11d show the interpolated energy assessment signal of an individual having abnormal heart function at E1, E2, E3, and E4, respectively. Comparison of the graphs of Fig. 10 with the corresponding graphs of Fig. 11 shows that the volume under the graph of the energy assessment at E1 and E2 is larger for the healthy individual (Fig. 10) than that of the unhealthy individual (Fig. 11). On the other hand, the volume under the graph of the energy assessment signal at E3 and E4 is smaller for the healthy individual than that of the unhealthy individual.

Graphs of the interpolated energy assessment signal, such as those shown in Figs. 10 and 11, can be displayed as a movie over one or more cardiac cycles for the diagnosis of heart disease. The volume under the graphs can be calculated at E1, E2, E3, and E4, and compared to predetermined thresholds. More preferably, however, the volume under the energy assessment signal at the various times can be calculated only over the apex of the heart, and compared to predetermined thresholds.

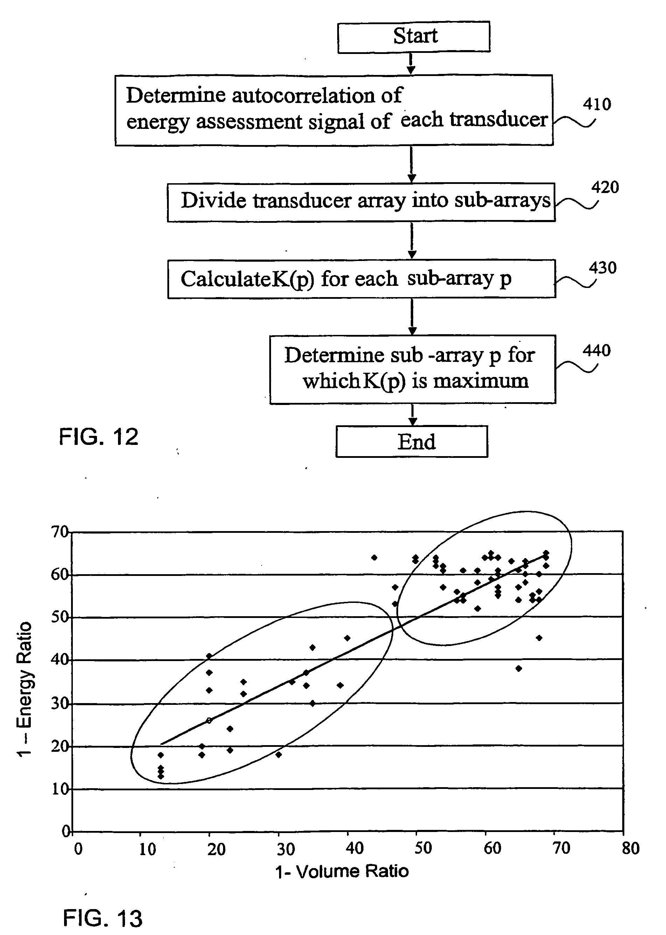

When a microphone array is used that covers a region of the body surface expending beyond the heart apex, the method shown in Fig. 12 may be used to identify the microphones in the array overlying the apex. In step **410**, the periodicity of the $\sigma_{fs}^{norm}(x_l, k_s)$ signals is determined by computing, for each transducer, the autocorrelation $P(xi, k_s)$ of the function $\sigma_{fs}^{norm}(x_l, k_s)$:

$$\hat{R}_{xy}(m) = \begin{cases} \sum_{n=0}^{N-m-1} x_{n+m} y_n^* & m \geq 0 \\ \hat{R}_{yx}^*(-m) & m < 0 \end{cases} \qquad (7)$$

where $\hat{R}_{xy}(m)$ is the autocorrelation, $n=k_s$, $x=y=P(xi, k_s)$ and $m$ is the shift value which varies from $-N$ to $N$ where N is the length of the vector $P(xi, k_s)$.

In step **420**, the transducer array is divided into possibly overlapping subarrays of adjacent transducers. For example, if the transducers are arranged in the array in a regular lattice, the transducer array may be divided into overlapping $n_r \times m_c$ rectangular or parallelogram subarrays of transducers. Then, in step **430**, for each subarray p of transducers, a parameter $K(p)$ is calculated, where

$$K(p) = \sum_{i \in p} \sum_{k_s} abs\left[diff\left(\hat{R}_{xy}(m)\right)\right] \qquad (8)$$

where the outer summation is taken over all transducers $i$ in the transducer subarray p. The subarray of microphones p for which $K(p)$ maximal is determined (step **440**). The subarray of transducers for which $K(p)$ maximal produces the best periodic sound signals, and since non-cardiac sounds have been filtered out of the signals or the subject did not breath during recording of the cardiovascular sounds, this subarray p of transducers produces the best cardiac sound signals, and thus consists of transducers overlying the heart apex.

The energy assessment signals shown in Fig. 8 were analyzed to find the transducers overlying the heart apex. The transducers were divided into subarrays of transducers. The square labeled "*Apex*" in Fig. 8 indicates a heart apex region array of transducers in the transducer array that was determined to lie over the heart apex by the method described above.

Example 3: Determination of Ejection Fraction.

In cardiovascular physiology, the term "*ejection fraction*" refers to the fraction of the blood introduced into a heart chamber during diastole that is pumped out of the chamber during systole. Although an ejection fraction can be calculated for any one of the four heart chambers, the left ventricular ejection fraction ("*LVEF*") is the ejection fraction that is most often determined in cardiological examinations.

Healthy individuals typically have left ventricular ejection fractions greater than 60%. Damage to the heart muscle, such as occurs in myocardial infarction or in cardiomyopathy, impairs the heart's ability to eject blood and therefore reduces the ejection fraction of one or more of the heart ventricles. This reduction in the ejection fraction can manifest itself clinically as heart failure. Ejection fraction is one of the most important predictors of prognosis. Those with significantly reduced ejection fractions typically have a poorer prognosis.

One common method for measuring ventricular ejection fraction, echocardiography, uses ultrasound images of the heart to obtain a sequence of 2- or 3- dimensional images of the heart over one or more heart cycles. The sequence of images is analyzed in order to calcuate a volume of a ventricle at the end of diastole when the ventricle has attained its maximum volume (the "*end diastolic volume*") and the volume of the same ventricle at the end of systole when it has attained its minimum volume. (the "*end systolic volume*"). The left ventricular ejection fraction (LVEF) is then obtained as the fraction of the end diastolic blood volume ejected from the left ventricle by the end of systole:

$$LVEF = 1 - \frac{\text{end systolic volume}}{\text{end diastolic volume}} \qquad (1)$$

Other methods for imaging the heart have also been used to determine ventricular volumes and to calculate ejection fraction. Such methods include pulsed or continuous wave Doppler ultrasound, cardiac magnetic resonance imaging (MRI), fast scan cardiac computed tomography (CT) imaging, and multiple gated acquisition (MUGA) scanning.

The energy assessment function can be used to determine left ventricular ejection fraction. This application of the invention is based on the novel and unexpected finding that the ratio $\frac{\text{Volume}_{(E3, E4)}}{\text{Volume}_{(E1, E2)}}$ (hereinafter referred to as the "*energy ratio*"), can be correlated with the ratio of the end systolic volume to the end diastolic volume, $\frac{\text{end systolic volume}}{\text{end diastolic volume}}$ (hereinafter referred to as the "*volume ratio*"), where Volume$_{(E3, E4)}$ is the maximum of the volume under the interpolated energy assessment signal over the heart apex at the time of E3 and E4, and Volume$_{(E1, E2)}$ is the volume under the interpolated energy assessment signal over the heart apex at time E1 or E2.

Energy ratios were determined in 32 subjects, as explained above. Volume ratios were obtained simultaneously by echocardiography. A total of 80 pairs of energy ration and volume ratio were obtained. Fig. 13 shows a scatter plot in which 1- the energy ratio of the 80 determinations is plotted as a function of the subject's ejection fraction (1- the volume ratio). The data points were subjected to a linear regression analysis, which produced the linear fit y=0.7884x+10.214, where y is the energy ratio and x is the volume ratio. The regression had a regression coefficient $r^2$=0.7706.

Fig. 13 shows that of the 80 points, 53 were indicative of a normal an ejection fraction (1- the volume ratio>0.5) and all but 2 of those 53 points also had a 1- the energy ratio of over 0.5. The other 27 points were indicative of an abnormal enection fraction (1- the volume ratio<0.5), and all but 3 of those 27 points had a 1-the energy ratio of below 0.5. Thus, the method of the invention classified 87.8% of the 80 points as normal or abnormal consistently with the echocardiography.

The ejection fraction can be calculated for each of two or more, and preferably, at least three, cardiac cycles in the energy signal by the method described above and the two or more calculated ejection fractions averaged together.

**Claims**

1. A system for analyzing body sounds from one or more body organs, comprising:

   (a) An integer N of transducers, each transducer configured to be fixed on a region of a surface of the individual, a transducer *i* being fixed at a location $x_i$ and generating a signal $S(x_i, t)$ indicative of pressure waves at the

location $x_i$; for i=1 to N;

(b) a processor receiving the signals $S(x_i,t)$ configured, for each of one or more of the signals $S(x_i,t)$ and for each of one or more time intervals k, to:

(i) calculate an average $\overline{S}_k$ of the signal $S(x_i,t)$ in the interval k; and
(ii) for one or more times $t_j$ in the interval k, calculate a difference $S(x_i,t_j)$- $\overline{S}k$; and
(iii) calculate an energy assessment signal in a calculation involving the one or more differences $S(x_i,t_j)$-$\overline{S}_k$.

**2.** The system according to Claim 1 wherein the processor is configured to filter from one or more of the signals $S(x_i,t)$ to remove one or more components of the signals $S(x_i,t)$ not arising from the body organ or organs.

**3.** The system according to Claim 1 or 2 wherein the calculation of the energy assessment signal involves calculation of a standard deviation signal from one or more of the signals $S(x_i,t)$.

**4.** The system according to Claim 3 wherein the standard deviation signal is obtained from the signal $S(x_i,t)$ or from a signal derived from the signal $S(x_i,t)$ in a process comprising dividing the filtered signal into time intervals by a time window and calculating the standard deviation $\sigma(k)$ for each interval using the algebraic expression

$$\sigma(k) = \left( \frac{1}{n} \sum_{j=1}^{n} \left( S\ (x_i,t_j) - \overline{S}_k \right)^2 \right)^{\frac{1}{2}}$$

where $k$ is an interval number, $t_j$ is a time sample in the interval, n is the number of samples in the interval, and $\overline{S}_k$ is the average value of the signal in the interval:

$$\overline{S}_k = \frac{1}{n} \sum_{j=1}^{n} S(x_i,t_j).$$

**5.** The system according to Claim 3 or 4 wherein the energy assessment signal is calculated as a sum of squares of components of the standard deviation signals, before or after normalisation, before or after median filtering, and before or after smoothing.

**6.** The system according to any one of the previous claims wherein the processor is further configured to interpolate the plurality of energy assessment signals to obtain energy assessment signals at one or more locations between transducers in the transducer array.

**7.** The system according to any one of Claims 1 to 6 configured to analyze cardiovascular sounds.

**8.** The system according to Claim 7, wherein the processor is further configured to identify one or more of events E1, E2, E3 and E4 of a cardiac cycle.

**9.** The system according to Claim 8 wherein the processor is further configured to identify one or more transducers overlying a heart apex.

**10.** The system according to Claim 9 wherein the processor is further configured to compare any one or more of the acoustic energies at any one or more of the cardiac cycle events E1, E2, E3, and E4 to a predetermined threshold.

**11.** The system according to Claim 10 wherein the processor is further configured to make a diagnosis of abnormal heart function based upon any one or more of the comparisons.

**12.** The system according to any one of the previous claims wherein the processor is further configured to calculate an ejection fraction from the energy assessment signal.

**13.** The system according to Claim 12 wherein the ejection fraction is calculated in a calcualtion involving a volume

ratio $\dfrac{\text{Volume}_{(E3, E4)}}{\text{Volume}_{(E1, E2)}}$ , wherein Volume$_{(E3,E4)}$ is an acoustic energy at E3 or E4, and Volume$_{(E1,E2)}$ is an acoustic energy at E1 or E2.

**14.** A method for analyzing body sounds from one or more body organs, comprising:

(a) affixing an integer N of transducers on a region of a surface of the individual, a transducer $i$ being fixed at a location $x_i$, each transducer and generating a signal $S(x_i,t)$ indicative of pressure waves at the location $x_i$; for i=1 to N; and
(b) for each of one or more of the signals $S(x_i,t)$ and for each of one or more time intervals k:

(i) calculating an average $\overline{S}_k$ of the signal $S(x_i,t)$ in the interval k; and
(ii) for one or more times $t_j$ in the interval k, calculating a difference $S(x_i,t_j)$- $\overline{S}k$; and
(iii) calculating an energy assessment signal in a calculation involving the one or more differences $S(x_i,t_j)$- $\overline{S}_k$.

**15.** The method according to Claim 14 wherein the standard deviation signal is obtained from the signal $S(x_i,t)$ or from a signal derived from the signal $S(x_i,t)$ in a process comprising dividing the filtered signal into time intervals by a time window and calculating the standard deviation $\sigma(k)$ for each interval using the algebraic expression

$$\sigma(k) = \left( \frac{1}{n} \sum_{j=1}^{n} \left( S\,(x_i,t_j) - \overline{S}_k \right)^2 \right)^{\frac{1}{2}}$$

where $k$ is an interval number, $t_j$ is a time sample in the interval, n is the number of samples in the interval, and $\overline{S}_k$ is the average value of the signal in the interval:

$$\overline{S}_k = \frac{1}{n} \sum_{j=1}^{n} S(x_i,t_j).$$

**16.** The method according to Claim 14 or 15 further comprising calculating an ejection fraction in a calculation involving a volume ratio $\dfrac{\text{Volume}_{(E3, E4)}}{\text{Volume}_{(E1, E2)}}$ , wherein Volume$_{(E3, E4)}$ is an acoustic energy at E3 or E4, and Volume$_{(E1, E2)}$ is an acoustic energy at E1 or E2.

**17.** A computer program comprising computer program code means for performing all the steps of Claim 14 or 15 when said program is run on a computer.

**18.** A computer program as claimed in Claim 16 embodied on a computer readable medium.

**Patentansprüche**

**1.** Ein System zur Analyse von Körperschall aus einem oder mehreren Körperorganen enthaltend:

(a) Eine ganze Zahl N an Tranducern, jeder Transducer ist konfiguriert, auf einem Bereich einer Oberfläche eines Individuums befestigt zu werden, wobei ein Transducer $i$ an der Stelle $x_i$ befestigt ist und ein Signal $S(x_i, t)$ erzeugt, hinweisend für Druckwellen an der Stelle $x_i$; für i=1 bis N;
(b) einen Prozessor, der die Signale $S(x_i,t)$ empfängt, konfiguriert, jedes des einen oder mehreren der Signale $S(x_i,t)$ und für jedes des einen oder mehreren Zeitintervalle k:

(i) einen Durchschnitt $\overline{S}_k$ des Signals $S(x_i,t)$ in dem Intervall k zu berechnen; und

(ii) für eine oder mehrere Zeiten $t_j$ in dem Intervall **k**, eine Differenz $S(x_i,t_j)$- $\overline{S}_k$ zu berechnen; und
(iii) ein Energiebewertungssignal in einer Berechnung, die eine oder mehrere Differenzen $S(x_i,t_j)$- $\overline{S}_k$ involviert, zu berechnen.

**2.** Das System nach Anspruch 1, wobei der Prozessor konfiguriert ist, aus einem oder mehreren der Signale $S(x_i,t)$ zu filtern, um eine oder mehrere Komponenten des Signals $S(x_i,t)$ zu entfernen, die nicht aus einem Körperorgan oder Organen entstehen.

**3.** Das System nach Anspruch 1 order 2, wobei die Berechnung des Energiebewertungssignals die Berechnung eines Standardabweichungssignals aus einem oder mehreren der Signale $S(x_i,t)$ involviert.

**4.** Das System nach Anspruch 3, wobei das Standardabweichungssignal aus dem Signal $S(x_i,t)$ erhalten wird oder aus einem Signal abgeleitet aus dem Signal $S(x_i,t)$ in einem Verfahren enthaltend Aufteilen des gefilterten Signals in Zeitintervalle durch ein Zeitfenster und Berechnen der Standardabweichung $\sigma(k)$ für jedes Intervall unter Verwendung des algebraischen Ausdrucks

$$\sigma(k) = \left( \frac{1}{n} \sum_{j=1}^{n} \left( S(x_i,t_j) - \overline{S}_k \right)^2 \right)^{\frac{1}{2}},$$

wobei k eine Intervallzahl ist, $t_j$ eine Zeitprobe in dem Intervall ist, n die Anzahl der Proben in dem Intervall ist und $\overline{S}_k$ der Durchschnittswert des Signals in dem Intervall ist:

$$\overline{S}_k = \frac{1}{n} \sum_{j=1}^{n} S(x_i,t_j).$$

**5.** Das System nach Anspruch 3 oder 4, wobei das Energiebewertungssignal berechnet wird als eine Summe der Quadrate der Komponenten der Standardabweichungssignale vor oder nach Normalisieren, vor oder nach Medianfiltern und vor oder nach Glätten.

**6.** Das System nach einem der vorhergehenden Ansprüche, wobei der Prozessor weiter konfiguriert ist, die Mehrheit des Energiebewertungssignals zu interpolieren, um Energiebewertungssignale an einer oder mehreren Stellen zwischen den Transducern in dem Transducer Array zu erhalten.

**7.** Das System nach einem der Ansprüche 1 bis 6, konfiguriert, um kardiovaskulären Schall zu analysieren.

**8.** Das System nach Anspruch 7, wobei der Prozessor weiter konfiguriert ist, ein oder mehrerer Ereignisse E1, E2, E3 und E4 eines Herzzyklus zu identifizieren.

**9.** Das System nach Anspruch 8, wobei der Prozessor weiter konfiguriert ist, einen oder mehrere Transducer, die über einer Herzspitze liegen, zu identifizieren.

**10.** Das System nach Anspruch 9, wobei der Prozessor weiter konfiguriert ist, einen oder mehrere der akustischen Energien an einem oder mehreren der Herzzyklusereignisse E1, E2, E3 und E4 an einer vorherbestimmten Schwelle zu vergleichen.

**11.** Das System nach Anspruch 10, wobei der Prozessor weiter konfiguriert ist, eine Diagnose einer anormalen Herzfunktion basierend auf einem oder mehreren der Vergleiche zu stellen.

**12.** Das System nach einem der vorhergehenden Ansprüche, wobei der Prozessor weiter konfiguriert ist, eine Auswurffraktion aus dem Energiebewertungssignal zu berechnen.

**13.** Das System nach Anspruch 12, wobei die Auswurffraktion berechnet ist in einer Berechnung, die ein Volumenver-

hältnis $\dfrac{\text{Volumen}_{(E3,\,E4)}}{\text{Volumen}_{(E1,\,E2)}}$ beinhaltet, wobei Volumen $_{(E3,\,E4)}$ eine akustische Energie bei E3 oder E4 und Volumen $_{(E1,\,E2)}$ eine akustische Energie bei E1 oder E2 ist.

**14.** Ein Verfahren zur Analyse von Körperschall aus einem oder mehreren Körperorganen enthaltend:

(a) Anheften einer ganzen Zahl N von Transducer an einen Bereich auf einer Oberfläche eines Individuums, wobei ein Transducer *i* an einer Stelle $x_i$ befestigt ist, und jeder Transducer ein Signal $S(x_i,t)$ erzeugt, hinweisend auf Druckwellen an der Stelle $x_i$; für i=1 bis N; und
(b) für jedes des einen oder mehreren Signals $S(x_i,t)$ und für jedes des einen oder mehreren Zeitintervalle k;

(i) Berechnen eines Durchschnitts $\overline{S}_k$ des Signals $S(x_i,t)$ in dem Intervall k; und
(ii) für ein oder mehrere Zeiten $t_j$ in dem Intervall k Berechnen einer Differenz $S(x_t, t_j)\text{-}\overline{S}_k$; und
(iii) Berechnen eines Energiebewertungssignals in einer Berechnung, die ein oder mehrere Differenzen $S(x_i,t_j)\text{-}\overline{S}_k$ involviert.

**15.** Das Verfahren nach Anspruch 14, wobei das Standardabweichungssignal erhalten wird aus dem Signal $S(x_i,t)$ oder aus einem Signal abgeleitet aus dem Signal $S(x_i,t)$ in einem Verfahren enthaltend Aufteilen des gefilterten Signals in Zeitintervalle durch ein Zeitfenster und Berechnen der Standardabweichung $\sigma(k)$ für jedes Intervall unter Verwendung des algebraischen Ausdrucks

$$\sigma(k) = \left( \frac{1}{n} \sum_{j=1}^{n} \left( S\ (x_i, t_j) - \overline{S}_k \right)^2 \right)^{\frac{1}{2}},$$

wobei k eine Intervallzahl ist, $t_j$ eine Zeitprobe in dem Intervall ist, n die Anzahl der Proben in dem Intervall ist und $\overline{S}_k$ der Durchschnittswert des Signals in dem Intervall ist:

$$\overline{S}_k = \frac{1}{n} \sum_{j=1}^{n} S(x_i, t_j).$$

**16.** Das Verfahren nach Anspruch 14 oder 15, weiter enthaltend Berechnen einer Auswurffraktion in einer Berechnung, die ein Volumenverhältnis $\dfrac{\text{Volumen}_{(E3,\,E4)}}{\text{Volumen}_{(E1,\,E2)}}$ beinhaltet, wobei Volumen $_{(E13,\,E4)}$ eine akustische Energie bei E3 oder E4 und Volumen $_{(E1,\,E2)}$ eine akustische Energie bei E1 oder E2 ist.

**17.** Ein Computerprogramm enthaltend ein Computerprogrammcodemittel zur Durchführung aller Schritte von Anspruch 14 oder 15, wenn das Programm auf einem Computer abläuft.

**18.** Ein Computerprogramm wie in Anspruch 16 beansprucht, verkörpert auf einem computerlesbaren Medium.

**Revendications**

**1.** Système d'analyse de sons corporels provenant d'un ou de plusieurs organes corporels, comprenant :

(a) un nombre entier N de transducteurs, chaque transducteur étant configuré pour être fixé sur une région d'une surface de l'individu, un transducteur i étant fixé à un emplacement $x_i$ et générant un signal $S(x_i,t)$ indicatif d'ondes de pression à l'emplacement $x_i$ ; pour i = 1 à N ;
(b) un processeur recevant les signaux $S(x_i,t)$ configuré, pour chacun d'un ou de plusieurs des signaux $S(x_i,t)$ et pour chacun d'un ou de plusieurs intervalles de temps k, pour :

(i) calculer une moyenne $\overline{S}_k$ du signal $S(x_i,t)$ dans l'intervalle k ; et

(ii) pour un ou plusieurs instants $t_j$ dans l'intervalle k, calculer une différence $S(x_i, t_j) - \overline{S}_k$ ; et

(iii) calculer un signal d'estimation d'énergie dans un calcul impliquant lesdites une ou plusieurs différences $S(x_i,t_j) - \overline{S}_k$.

2. Système selon la revendication 1, dans lequel le processeur est configuré pour filtrer un ou plusieurs des signaux $S(x_i,t)$ pour retirer une ou plusieurs composantes des signaux $S(x_i,t)$ ne provenant pas de l'organe ou des organes corporels.

3. Système selon la revendication 1 ou 2, dans lequel le calcul du signal d'estimation d'énergie implique le calcul d'un signal d'écart type à partir d'un ou de plusieurs des signaux $S(x_i,t)$.

4. Système selon la revendication 3, dans lequel le signal d'écart type est obtenu à partir du signal $S(x_i,t)$ ou à partir d'un signal déduit du signal $S(x_i,t)$ dans un processus comprenant la division du signal filtré en intervalles de temps par une fenêtre de temps et le calcul de l'écart type $\sigma(k)$ pour chaque intervalle en utilisant l'expression algébrique

$$\sigma(k) = \left( \frac{1}{n} \sum_{j=1}^{n} \left( S(x_i, t_j) - \overline{S}_k \right)^2 \right)^{\frac{1}{2}}$$

où k est un nombre d'intervalles, $t_j$ est un échantillon de temps dans l'intervalle, n est le nombre d'échantillons dans l'intervalle, et $\overline{S}_k$ est la valeur moyenne du signal dans l'intervalle :

$$\overline{S}_k = \frac{1}{n} \sum_{j=1}^{n} S(x_i, t_j).$$

5. Système selon la revendication 3 ou 4, dans lequel le signal d'estimation d'énergie est calculé en tant que somme des carrés des composantes des signaux d'écart type, avant ou après une normalisation, avant ou après un filtrage médian, et avant ou après un lissage.

6. Système selon l'une quelconque des revendications précédentes, dans lequel le processeur est en outre configuré pour interpoler la pluralité de signaux d'estimation d'énergie pour obtenir des signaux d'estimation d'énergie à un ou plusieurs emplacements entre des transducteurs dans l'ensemble de transducteurs.

7. Système selon l'une quelconque des revendications 1 à 6, configuré pour analyser des sons cardiovasculaires.

8. Système selon la revendication 7, dans lequel le processeur est en outre configuré pour identifier un ou plusieurs événements E1, E2, E3 et E4 d'un cycle cardiaque.

9. Système selon la revendication 8, dans lequel le processeur est en outre configuré pour identifier un ou plusieurs transducteurs recouvrant un apex du coeur.

10. Système selon la revendication 9, dans lequel le processeur est en outre configuré pour comparer l'une quelconque ou plusieurs des énergies acoustiques lors de l'un quelconque ou de plusieurs des événements de cycle cardiaque E1; E2, E3 et E4 à un seuil prédéterminé.

11. Système selon la revendication 10, dans lequel le processeur est en outre configuré pour diagnostiquer une fonction cardiaque anormale sur la base de l'une quelconque ou de plusieurs des comparaisons.

12. Système selon l'une quelconque des revendications précédentes, dans lequel le processeur est en outre configuré pour calculer une fraction d'éjection à partir du signal d'estimation d'énergie.

13. Système selon la revendication 12, dans lequel la fraction d'éjection est calculée dans un calcul impliquant un rapport de volume Volume$_{(E3,E4)}$/Volume$_{(E1,E2)}$, où Volume$_{(E3,E4)}$ est une énergie acoustique pour E3 ou E4 et Volume$_{(E1,E2)}$ est une énergie acoustique pour E1 ou E2.

14. Procédé d'analyse de sons corporels provenant d'un ou de plusieurs organes corporels, consistant à :

   (a) apposer un nombre entier N de transducteurs sur une région d'une surface de l'individu, un transducteur i étant fixé à un emplacement $x_i$ et générant un signal $S(x_i,t)$ indicatif d'ondes de pression à l'emplacement $x_i$ ; pour i = 1 à N ; et
   (b) pour chacun d'un ou de plusieurs des signaux $S(x_i,t)$ et pour chacun d'un ou de plusieurs intervalles de temps k :

   (i) calculer une moyenne $\overline{S}_k$ du signal $S(x_i,t)$ dans l'intervalle k ; et
   (ii) pour un ou plusieurs instants $t_j$ dans l'intervalle k, calculer une différence $S(x_i,t_j) - \overline{S}_k$ ; et
   (iii) calculer un signal d'estimation d'énergie dans un calcul impliquant lesdites une ou plusieurs différences $S(x_i,tj) - \overline{S}_k$.

15. Procédé selon la revendication 14, dans lequel le signal d'écart type est obtenu à partir du signal $S(x_i,t)$ ou à partir d'un signal déduit du signal $S(x_i,t)$ dans un processus comprenant la division du signal filtré en intervalles de temps par une fenêtre de temps et le calcul de l'écart type $\sigma(k)$ pour chaque intervalle en utilisant l'expression algébrique

$$\sigma(k) = \left( \frac{1}{n} \sum_{j=1}^{n} \left( S(x_i, t_j) - \overline{\overline{S}}_k \right)^2 \right)^{\frac{1}{2}}$$

où k est un nombre d'intervalles, $t_j$ est un échantillon de temps dans l'intervalle, n est le nombre d'échantillons dans l'intervalle, et $\overline{S}_k$ est la valeur moyenne du signal dans l'intervalle :

$$\overline{S}_k = \frac{1}{n} \sum_{j=1}^{n} S(x_i, t_j).$$

16. Procédé selon la revendication 14 ou 15, comprenant en outre le calcul d'une fraction d'éjection dans un calcul impliquant un rapport de volume Volume$_{(E3,E4)}$/Volume$_{(E1,E2)}$, où Volume$_{(E3,E4)}$ est une énergie acoustique pour E3 ou E4 et Volume$_{(E1,E2)}$ est une énergie acoustique pour E1 ou E2.

17. Programme d'ordinateur comprenant des moyens formant code de programme d'ordinateur pour effectuer toutes les étapes de la revendication 14 ou 15 lorsque ledit programme est exécuté sur un ordinateur.

18. Programme d'ordinateur selon la revendication 16 mis en oeuvre sur un support pouvant être lu par un ordinateur.

EP 2 120 720 B1

FIG. 1

FIG. 2

**FIG. 4**

Start → Divide signal into subintervals (320) → Calculate sum of squares in each subinterval (325) → Interpolate (330) → End

**FIG. 3**

Start → Calculate standard deviation signals $\sigma(x_i,k)$, $k=1\ldots n_k$ (215) → Normalize standard deviation signals $\sigma^{norm}(x_i,k)$, $k=1\ldots n_k$ (216) → Filter $\sigma^{norm}(x_i,k)$, $k=1\ldots n_k$ (217) → Perform extended smoothing on $\sigma_f^{norm}(x_i,k)$ (220) → Calculate energy Assessment signal (225) → End

FIG.5a

FIG.5b

FIG.5c

EP 2 120 720 B1

FIG. 7b

ECG

FIG. 7a

Acoustic signal

FIG. 7c

Acoustic Signal Envelop

msec

FIG. 6b

ECG

FIG. 6a

Acoustic signal

FIG. 6c

Acoustic Signal Envelop

msec

18

FIG.8

Normal

Apex

Base

Spine

Frame # 344

90

FIG. 9a

Abnormal

Apex

Base

Spine

Frame # 360

91

FIG. 9b

FIG. 10a

FIG. 10b

FIG. 10c

FIG. 10d

FIG. 11a

FIG. 11b

FIG. 11c

FIG. 11d

24

Start

| Determine autocorrelation of energy assessment signal of each transducer | 410 |

| Divide transducer array into sub-arrays | 420 |

| Calculate K(p) for each sub-array p | 430 |

| Determine sub-array p for which K(p) is maximum | 440 |

End

**FIG. 12**

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6139505 A
- US 6887208 A

- WO 2004105612 A

**Non-patent literature cited in the description**

- **KOMPIS et al.** *Chest,* 2001, vol. 120 (4